Europäisches Patentamt

European Patent Office

Office européen des brevets

(11) Veröffentlichungsnummer: **0 169 972**
**B1**

(12) **EUROPÄISCHE PATENTSCHRIFT**

(45) Veröffentlichungstag der Patentschrift:
30.08.89

(51) Int. Cl.⁴: **A 61 M 13/00**

(21) Anmeldenummer: 85104002.2

(22) Anmeldetag: 02.04.85

(54) Vorrichtung zum Insufflieren von Gas in den Körper.

(30) Priorität: 11.04.84 DE 3413631

(43) Veröffentlichungstag der Anmeldung:
05.02.86 Patentblatt 86/06

(45) Bekanntmachung des Hinweises auf die Patenterteilung: 30.08.89 Patentblatt 89/35

(84) Benannte Vertragsstaaten:
DE FR GB NL

(56) Entgegenhaltungen:
DE—A— 2 611 698
DE—A— 2 803 646

(73) Patentinhaber: Semm, Kurt, Prof. Dr.
Hegewischstrasse 4
D-2300 Kiel (DE)

(72) Erfinder: Semm, Kurt, Prof. Dr.
Hegewischstrasse 4
D-2300 Kiel (DE)
Erfinder: Neumann, Peter, Dipl.-Phys.
Landshuterstrasse 8
DE-8301 Furth (DE)

(74) Vertreter: Weber, Otto Ernst, Dipl.-Phys.
Hofbrunnstrasse 36
D-8000 München 71 (DE)

## Beschreibung

Die Erfindung betrifft ein Verfahren zur Messung des intraabdominellen Druckes beim Insufflieren von Gas in den intraabdominalen Bereich eines menschlichen oder tierischen Körpers gemäß Oberbegriff des Anspruches 1 sowie eine Vorrichtung zur Durchführung des Verfahrens gemäß Anspruch 2.

Ein derartiges Verfahren ist beispielsweise aus der DE-A-28 03 646 bekannt und dient dort dazu, insbesondere den Bauchraum kontrolliert mit Gas zu füllen, so daß endoskopische Arbeiten ermöglicht bzw. erleichtert werden. Dabei ist es erforderlich, den Druck in Bauchraum innerhalb physiologisch vertretbarer Grenzen konstant zu halten und genau zu messen. Eine Vorrichtung zur Durchführung des vorgenannten Verfahrens ist aus der DE-A-28 03 646 bzw. aus der DE-A-26 11 698 bekannt. Bei diesen Vorrichtungen erfolgt die Zufuhr des Gases in den Bauchraum und die Messung des intraabdominalen statischen Druckes über ein einziges Rohr. Das Insufflieren des Gases erfolgt dabei intermittierend, wobei in den Strömungspausen der Gasfluß gestoppt wird und der intraabdominale Druck direkt gemessen wird. Dieses Verfahren bzw. diese Vorrichtung hat sich zwar bei verschiedenen Operationsbedingungen gut bewährt. Es sind jedoch noch Nachteile vorhanden. So hat z.B. der Operateur während des Einströmens des Gases in den Bauchraum keinerlei Kenntnis über den tatsächlichen Druck im Abdomen. Da beim genannten intermittierenden Betrieb die Einblaszeiten wesentlich länger sind als die Zeitspannen, in denen der Gasfluß gestoppt und der Druck gemessen wird, hat der Arzt über relativ weite Zeitspannen keinen Überblick über den aktuellen statischen intraabdominellen Druck.

Der vorliegenden Erfindung liegt daher die Aufgabe zugrunde, ein Verfahren zum Insufflieren von Gas in den abdominalen Bereich des menschlichen oder tierischen Körpers und eine Vorrichtung zur Durchführung des Verfahrens zu schaffen, wobei Verfahren und Vorrichtung dem Arzt jederzeit Kenntnis über den tatsächlichen statischen intraabdominellen Druck vermitteln sollen.

Diese Aufgabe wird verfahrensmäßig durch die Merkmale des kennzeichnenden Teils des Anspruchs 1 gelöst. Vorrichtungsmäßig wird dies durch die Merkmale des kennzeichnenden Teils des Anspruchs 2 gelöst.

Die Erfindung macht sich einerseits die Erkenntnis zunutze, daß die Strömungsgeschwindigkeit in einer Leitung vom an beiden Seiten der Leitung herrschenden Druckgefälle abhängt. So herrscht bei einem Insufflationsapparat auf der einen Seite der Leitung der gegebenenfalls durch Druckminderer reduzierte Druck eines Zwischenbehälters (typischerweise 40 mm Hg), während auf der anderen Seite der Leitung, also im Bereich des Insufflationsinstrumentes (Veress-Nadel) ein niedrigerer Druck herrscht, nämlich der intraabdominale statische Druck. Die Strömungsgeschwindigkeit des Gases in der Leitung ist eine Funktion der genannten Druckdifferenz und da der höhere Druck im Anschluß an den Zwischenspeicher und den Druckminderer grundsätzlich feststellbar bzw. bekannt ist, ergibt sich aus dieser Funktion der zu messende intraabdominale statische Druck.

Ein derartiger Schritt zur Messung des intraabdominalen Druckes mittels einer reinen Strömungsgeschwindigkeits-Messung bringt jedoch Unsicherheiten mit sich, da Messungen der Strömungsgeschwindigkeit technisch relativ unsicher sind. Außerdem ist zu berücksichtigen, daß bei den hier in Rede stehenden Insufflationsapparaten die Veress-Nadel eine relativ kleinlumige Düse aufweist. Auch sind die Strömungsverhältnisse in dem Körperinneren unmittelbar im Bereich der Veress-Nadel bei jedem Einstich verschieden. Hinzu kommt, daß das gesamte Leitungssystem vom Druckminderer bis zum Insufflationsinstrument in der täglichen Praxis des Operationsbetriebes in der Regel nicht konstant gehalten werden kann, da Schläuche etc. umgelegt und verändert werden. Diese Umstände haben zur Folge, daß in der Praxis der Strömungswiderstand des Leitungssystems — wobei zum Leitungssystem auch die unmittelbare Umgebung der Veress-Nadel hinzugezählt werden muß — von Operation zu Operation unterschiedlich sein kann. Bei einer reinen Messung der Strömungsgeschwindigkeit können daher diese wechselnden Umstände nur schwerlich ausreichend berücksichtigt werden.

Deshalb wird der vorgenannte Schritt durch die Berücksichtigung des Strömungs- bzw. Leitungswiderstandes ergänzt. Man mißt daher den statischen Druck im Leitungssystem vor dem Insufflationsinstrument. Die Strömungsgeschwindigkeit des Gases wird dabei mittels eines Durchflußreglers auf einen konstanten vorgebbaren Wert geregelt. Diesen Maßnahmen liegt die Erkenntnis zugrunde, daß der so gemessene Druck vor dem Insufflationsinstrument eine Funktion des zu messenden intraabdominalen Druckes ist, so daß er sich mittels einer elektronischen Auswerteschaltung entsprechend umrechnen läßt.

Aufgrund der oben beschriebenen wechselnden Umstände von Operation zu Operation ist es aber in der Regel erforderlich, in jedem Einzelfall den tatsächlichen Strömungswiderstand des Leitungssystems sowie des Austrittsbereichs der Veress-Nadel unmittelbar zu messen. Diese Messung des im Einzelfall vorliegenden Strömungswiderstandes des Leitungssystems, welcher hauptsächlich durch die Veress-Nadel bedingt ist, erfolgt dadurch, daß der in die elektronische Auswerteschaltung eingegebene Meßwert des Druckmeßgerätes nach der Zeit differenziert wird und daß derjenige Druckwert, welcher bei Beginn des Insufflierens in dem Zeitpunkt gemessen wird, in dem die erste Ableitung des Meßwerts gleich 0 ist, als der Vorrichtung zuzuordnender Widerstandsdruck der Leitung und des Insufflationsinstrumentes gewählt wird.

Hierbei macht man sich die Erkenntnis zunutze, daß bei Beginn des Insufflierens, also beim Öffnen der Gaszufuhreinrichtungen, zunächst sehr instabile Verhältnisse herrschen, d.h. der Meßwert des Druckmeßgerätes schwankt zunächst relativ heftig. Kurz darauf stellen sich aber stationäre Bedingungen ein, so daß der Meßwert des Druckmeßgerätes in etwa konstant ist. Wird demzufolge der Meßwert des Druckmeßgerätes nach der Zeit differenziert, so wird die erste Ableitung unmittelbar mit dem Einstellen stationärer Strömungsverhältnisse gleich 0. Zu diesem Zeitpunkt ist aber im Bauchraum noch kein nennenswerter intraabdominaler Überdruck aufgebaut. Der vor dem Insufflationsinstrument am Druckmeßgerät anstehende Druck ist somit zu diesem Zeitpunkt allein durch den Strömungswiderstand des Leitungssystems, insbesondere des Insufflationsinstruments, bedingt. Der auf diese Weise zu Beginn des Insufflierens gemessene Druckwert ist also eine Apparatekonstante, bei welcher zugleich auch die Strömungsbedingungen in unmittelbarer Umgebung des Insufflationsinstrumentes berücksichtigt sind. Im weiteren Verlauf des Insufflierens nach den soeben geschilderten Anfangsverhältnissen steigt dann der Meßwert des Druckmeßgerätes allmählich an. Wird der bei Beginn des Insufflierens beim Einstellen stationärer Strömungsverhältnisse gemessene Druck am Druckmeßgerät mit $P_{wid}$ bezeichnet und der danach beim fortschreitenden Insufflieren gemessene, ansteigende Druck am Druckmeßgerät mit $P_{ges}$ (Widerstandsdruck bzw. Gesamtdruck), so ergibt sich der zu messende statische intraabdominale Druck höchst einfach durch Subtraktion des Wertes $P_{wid}$ von $P_{ges}$.

In den Unteransprüchen 3 bis 9 sind vorteilhafte Weiterbildungen der Erfindung beschrieben.

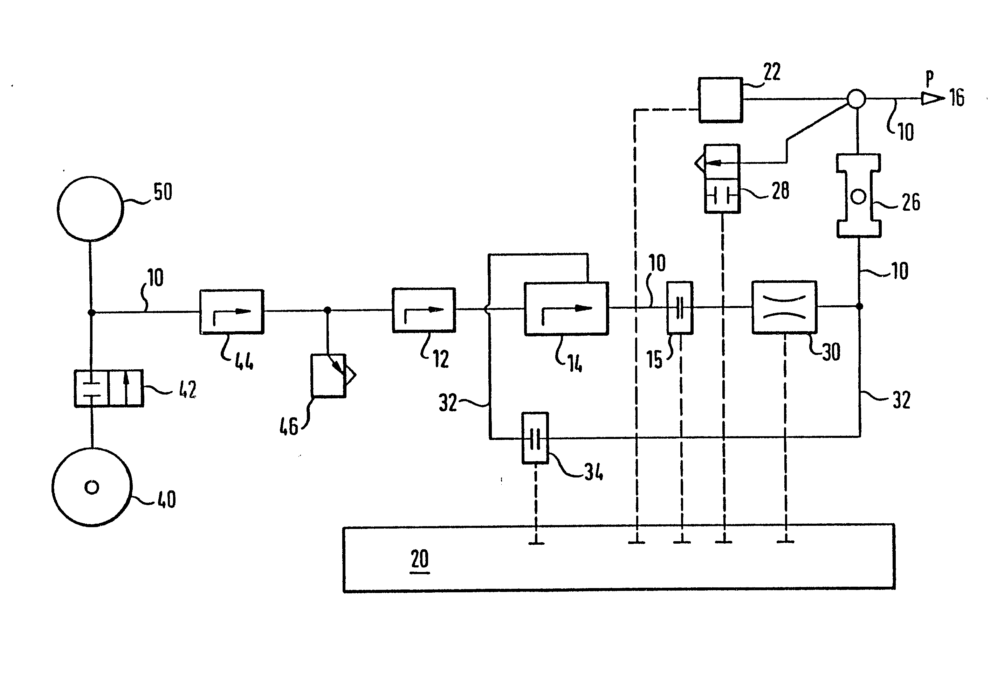

Nachfolgend ist die Erfindung anhand der Zeichnung im einzelnen erläutert. Dabei zeigt die Figur schematisch ein Ausführungsbeispiel einer erfindungsgemäßen Vorrichtung.

Eine Vorratsflasche 40 mit Kohlendioxid-Gas wird mittels eines Ventils 42 geöffnet. In einem Zwischenspeicher 50 wird Kohlendioxid-Gas gespeichert. Ein erster Druckminderer 44 reduziert den hohen Gasdruck im Zwischenspeicher 50 auf beispielsweise 0,85 bar. Ein Sicherheitsventil 46 öffnet, sobald der Druckminderer 44 versagt. Ein weiterer Druckminderer 12 reduziert den in der Leitung 10 herrschenden Druck auf beispielsweise 40 oder 50 mm Hg.

Stromab des Druckminderers 12 ist in der Leitung 10 ein Durchflußregler 14 angeordnet, welcher die in pro Zeiteinheit durchströmende Gasmenge auf einen festen, vorgebbaren Wert regelt. Weiterhin ist stromab des Durchflußreglers 14 ein Magnetventil 15 in der Leitung 10 angeordnet, mittels welchem der Gasstrom zum Patienten unterbrechbar ist.

Ein Durchflußmesser 30 mißt den tatsächlich fließenden Gasstrom und gibt den Meßwert in die elektronische Auswerteschaltung 20 ein. Der optischen Kontrolle des Gasstromes dient ein stromab des Durchflußmessers 30 in der Leitung 10 angeordneter Durchflußanzeiger 26. Im Anschluß an den Durchflußanzeiger 26 führt die Leitung 10 direkt zum Insufflationsinstrument, das im einzelnen nicht gezeigt ist und mit dem Bezugszeichen 16 symbolisch angedeutet ist. Der Pfeil P in der Figur weist also direkt zum Insufflationsinstrument. Weiterhin ist unmittelbar vor dem Insufflationsinstrument an der Leitung 10 ein Sicherheitsventil 28 angeordnet, welches bei Überschreiten eines vorgebbaren Maximaldruckes von beispielsweise 40 mm Hg öffnet. Wesentlich für die Erfindung ist das in der Leitung 10 vor dem Insufflationsinstrument 16 angeordnete Druckmeßgerät 22.

Neben der Haupt-Leitung 10 ist in der Vorrichtung noch eine Zweigleitung 32 vorgesehen, welche einen Punkt in der Leitung 10 stromauf des Durchflußreglers 14 mit einem Punkt stromab des Durchflußmeßgerätes 30 verbindet. Ein elektronisch betätigbares Ventil 34, wie beispielsweise ein Magnetventil dient dem Absperren der Leitung 32.

Das Zweigleitungsventil 34, das Hauptleitungsventil 15, der Durchflußmesser 30, das Sicherheitsventil 28 und das Druckmeßgerät 22 sind jeweils mit der elektronischen Auswerteschaltung 20 verbunden. An der elektronischen Auswerteschaltung 20 ist eine Anzeigevorrichtung (Display) vorgesehen, in welcher der vom Druckmeßgerät 22 gemessene Gesamtdruck, der ermittelte intraabdominale statische Druck, die Gasströmungsgeschwindigkeit sowie die insgesamt verbrauchte Gasmenge angezeigt werden.

Im einzelnen funktioniert die in der Figur gezeigte Vorrichtung wie folgt: Nach Öffnen des Absperrventils 42 der $CO_2$-Vorratsflasche 40 strömt das Gas in den Zwischenspeicher 50. Ein erster Druckminderer 44 in der Leitung reduziert den Druck auf beispielsweise 0,85 bar. Übersteigt der stromab des Druckminderers 44 in der Leitung anstehende Druck einen vorgegebenen Wert, so öffnet das Sicherheitsventil 46. Ein weiterer Druckminderer 12 reduziert den stromab von ihm anstehenden Druck auf z. B. 50 mm Hg. Der nachfolgende Durchflußregler regelt die Strömungsgeschwindigkeit des Gases in der Leitung 10 auf beispielsweise 1 Liter pro Minute. Das Gas strömt dann durch das Magnetventil 15 und den Durchflußmesser 30. Der am Ventil 15 und dem Durchflußmesser 30 infolge des Reibungswiderstandes der Leitung entstehende Druckabfall wird, da er immer einem bestimmten Gasfluß zuzuordnen ist, mit Hilfe des Durchflußreglers 14 konstant gehalten. Dieser Druckabfall dient der Durchflußregelung im Durchflußregler 14.

Nachdem das Gas sodann durch die optische Durchflußanzeigeeinrichtung 26 geströmt ist, gelangt es über eine Schlauchleitung und das Insufflationsinstrument (nicht gezeigt) zum Patienten. Die Schlauchleitung zum Patienten ist durch den Pfeil P angedeutet.

Beim Einschalten der Vorrichtung, d. h. beim Öffnen des Flaschenventils 42 wird nun der Anfangs-Druckanstieg am Druckmeßgerät 22 gemessen und der Meßwert in die elektronische Auswerteschaltung 20 eingegeben. Dort wird der Meßwert als Funktion der Zeit differenziert. Zu Beginn des Gasflusses wird der Meßwert zumindest eine heftige Schwankung ausführen. Sobald aber stationäre Strömungsverhältnisse eingetreten sind, ändert sich der gemessene Druckwert nur geringfügig so daß die Ableitung der Funktion Druckmeßwert (Zeit) gegen 0 geht. Die elektronische Auswerteschaltung 20 registriert in diesem

3

Zeitpunkt den anstehenden Meßwert am Druckmeßgerät 22. Da sich zu diesem Zeitpunkt noch kein nennenswerter intraabdominaler statischer Druck im Bauchraum des Patienten aufgebaut hat, ist der zu Beginn der stationären Strömungsverhältnisse gemessene und in der elektronischen Auswerteschaltung in einem Analogspeicher gespeicherte Druckwert ein Maß für den Strömungswiderstand des gesamten Leitungssystems, welcher hauptsächlich durch den Querschnitt der Düse des Insufflationsinstrumentes definiert ist. Danach wird der Gasstrom in der Bauchhöhle einen Überdruck gegen den äußeren Atmosphärendruck erzeugen, welcher durch das Insufflationsinstrument zurück auch auf das Druckmeßgerät 22 wirkt. Demzufolge wird am Druckmeßgerät 22 nicht nur der erwähnte Widerstandsdruck gemessen, sondern auch der intraabdominale statische Druck.

Zur Bestimmung des intraabdominalen statischen Druckes wird also vom momentan am Druckmeßgerät 22 gemessenen Druckwert $P_{ges}$ der anfangs zu Beginn der Strömung festgestellte Widerstandsdruck $P_{wid}$ abgezogen. Die Differenz ist direkt der intraabdominale statische Druck.

Bei Erreichen eines vorgewählten Druckes $P_{ges}$ wird der Gasfluß automatisch durch die elektronische Auswerteschaltung gestoppt wobei das Ventil 15 und das Ventil 34 in der Zweigleitung 32 synchron schließen. Das synchrone Verschließen bzw. Öffnen der Ventile 15 und 34 hat zur Folge, daß auch nach dem Schließen des Ventiles 15 der unmittelbar vor dem Schließen am Durchflußregler 14 anstehende Druckwert erhalten bleibt.

Bei Erreichen eines bestimmten, vorgewählten Druckes von z. B. 40 mm Hg wird der momentane Durchfluß-Meßwert in der elektronischen Auswerteschaltung 20 gespeichert und kann zur Messung des intraabdominalen Druckes verwendet werden. Hierbei ergibt sich der intraabdominale statische Druck gemäß der Formel

$$P_{abd} = P_{max}(1 - \frac{\text{momentane Strömungsgeschwindigkeit}}{\text{(maximale Strömungsgeschwindigkeit}}),$$

wobei $P_{abd}$ der intraabdominale Druck, $P_{max}$ ein für das Gerät bekannter Meßwert eines im Gerät begrenzten Maximaldruckes und die momentane Strömungsgeschwindigkeit die tatsächlich zu dem gegebenen Zeitpunkt gemessene Strömungsrate ist, während die maximale Strömungsgeschwindigkeit ein gerätespezifischer Meßwert für den Gasdurchfluß ist, welcher dann gegeben ist, wenn zum ersten Mal $P_{max}$ erreicht ist.


**Patentansprüche**

1. Verfahren zur Messung des intraabdominalen Druckes beim Insufflieren von Gas, insbesondere Kohlendioxid, in den intraabdominalen Bereich eines menschlichen oder tierischen Körpers, wobei beim Insufflieren das Gas aus einer Druckgasspeicherung über einen oder mehrere Zwischenspeicherungsschritte und zumindest einer Druckminderung über eine Leitung, einen Durchflußregler und ein Insufflationsinstrument, z. B. eine Veress-Nadel, in den intraabdominalen Bereich des Körpers geleitet wird, dadurch gekennzeichnet, daß der nach der Druckminderung in der Leitung vorhandene Gasdruck und der Strömungswiderstand der Leitung gemessen werden, und daß aus der Strömungsgeschwindigkeit in der Leitung, aus dem Strömungswiderstand und dem Gasdruck der intraabdominale Druck berechnet wird.

2. Vorrichtung zur Durchführung des Verfahrens nach Anspruch 1, mit einem Druckgasspeicher (40) für das zu insufflierende Gas, mit vorzugsweise einem oder mehreren Zwischenspeicher(n) (50) mit mindestens einem Druckminderer (44) in der das Gas in den intraabdominalen Bereich führenden Leitung (10) und einem Insufflationsinstrument (bei 16), z. B. einer Veress-Nadel, wobei in der Leitung (10) zwischen dem Druckminderer (12) und dem Insufflationsinstrument (16) ein Durchflußregler (14) für das Gas vorgesehen ist, sowie stromab des Durchflußreglers (14) ein Druckmeßgerät (22), dessen Meßwert als Funktion der Zeit einer elektronischen Auswerteschaltung (20) zugeführt ist, dadurch gekennzeichnet, daß der in die elektronische Auswerteschaltung (20) eingegebene Meßwert $P_{ges}$ des Druckmeßgerätes (22) nach der Zeit differenziert wird, daß der der Vorrichtung zuzuordnende Widerstandsdruck der Leitung (10) und des Insufflationsinstrumentes (16) derjenige Druckwert $P_{wid}$ ist, welcher bei Beginn des Insufflierens in dem Zeitpunkt gemessen wird, in dem die erste Ableitung des Druckwertes gleich « 0 » ist, und daß der intraabdominale Druck $P_{abd}$ durch die Druckdifferenz $P_{ges}$-$P_{wid}$ bestimmbar ist.

3. Vorrichtung nach Anspruch 2, dadurch gekennzeichnet, daß in der Leitung (10) zwischen dem Druckminderer (12) und dem Insufflationsinstrument (16) zumindest ein Meßgerät (30) für die Gasströmungsgeschwindigkeit vorgesehen ist.

4. Vorrichtung nach Anspruch 3, dadurch gekennzeichnet, daß stromab des Durchflußreglers (14) ein elektronisch betätigbares Ventil (15), z. B. ein Magnetventil, vorgesehen ist.

5. Vorrichtung nach Anspruch 3 oder 4, dadurch gekennzeichnet, daß stromab des Durchflußreglers (14) eine Durchfluß-Anzeigeeinrichtung (26) für das strömende Gas in der Leitung (10) vorgesehen ist.

6. Vorrichtung nach einem der Ansprüche 3 bis 5, dadurch gekennzeichnet, daß stromab des Durchflußreglers (14) bzw. der Durchfluß-Anzeigeeinrichtung (26) ein Sicherheitsventil (28), z. B. ein Magnetventil, in der Leitung (10) vorgesehen ist.

7. Vorrichtung nach einem der Ansprüche 2 bis 6, dadurch gekennzeichnet, daß stromab des Durchflußreglers (14) in der Leitung (10) ein Durchflußmeßgerät (30) vorgesehen ist, dessen Meßwert ebenfalls in die elektronische Auswerteschaltung (20) eingegeben wird.

8. Vorrichtung nach Anspruch 7, dadurch gekennzeichnet, daß eine Zweigleitung (32) vorgesehen ist, welche in der Leitung (10) einen Punkt stromauf des Durchflußreglers (14) mit einem Punkt stromab des Durchfluß-Meßgerätes (30) verbindet, wobei der Durchfluß in der Zweigleitung (32) von einem elektronisch betätigbaren Ventil (34), z. B. ein Magnetventil, steuerbar ist.

9. Vorrichtung nach Anspruch 8, dadurch gekennzeichnet, daß das Ventil (34) in der Zweigleitung (32) automatisch und synchron mit dem Ventil (15) stromab des Durchflußreglers (14) schließt bzw. öffnet.

## Claims

1. Process for measuring the intraabdominal pressure during the insufflation of gas, particularly carbon dioxide, into the intraabdominal region of a humam or animal body, in which, during insufflation, the gas is passed from a pressurized gas reservoir via one or more intermediate storage stages and at least one pressure reduction by a pipe, a flow regulator and an insufflation instrument, e. g. a Veress needle, into the intraabdominal region of the body, characterized in that the gas pressure present in the line following pressure reduction and the flow resistance of the line are measured and that the intraabominal pressure is calculated from the flow rate in the line, the flow resistance and the gas pressure.

2. Apparatus for performing the process according to claim 1, with a pressurized gas reservoir (40) for the gas to be insufflated, with preferably one or more intermediate reservoirs (50), with at least one pressure reducer (44) in the line (10) passing the gas into the intraabdominal region and an insufflation instrument (at 16), e. g. a Veress needle, a flow regulator (14) for the gas being provided in line (10) between the pressure reducer (12) and the insufflation instrument (16), as well as downstream of the flow regulator (14) a pressure gauge (22), whose measured value is supplied as a function of time to an electronic evaluation circuit (20), characterized in that the measured value $P_{ges}$ of the pressure gauge (22) fed into the electronic evaluation circuit (20) is differentiated according to time, that the resistance pressure of line (10) and insufflation instrument (16) to be associated with the apparatus is the pressure value $P_{wid}$ measured at the start of insufflation at the time in which the first derivation of the pressure value is equal to 0 and that the intraabdominal pressure $P_{abd}$ can be determined by the pressure difference $P_{ges}-P_{wid}$.

3. Apparatus according to claim 2, characterized in that at least one measuring instrument (30) for the gas flow rates is provided in line (10) between pressure reducer (12) and insufflation instrument (16).

4. Apparatus according to claim 3, characterized in that downstream of the flow regulator (14) is provided an electronically operable valve (15), e. g. a solenoid valve.

5. Apparatus according to claims 3 or 4, characterized in that downstream of the flow regulator (14) is provided a flow indicator (26) in line (10) for the flowing gas.

6. Apparatus according to one of the claims 3 to 5, characterized in that a safety valve (28), e. g. a solenoid valve is provided in line (10) downstream of flow regulator (14) or/of flow indicator (26).

7. Apparatus according to one of the claims 2 to 6, characterized in that in line (10) downstream of flow regulator (14) is provided a flow meter (30), whose measured value is also fed into the electronic evaluation circuit (20).

8. Apparatus according to claim 7, characterized in that a branch line (32) is provided, which, in line (10), links a point upstream of flow regulator (14) to a point downstream of flow meter (30), the flow in branch line (32) being controllable by an electronically operable valve (34), e. g. a solenoid valve.

9. Apparatus according to claim 8, characterized in that the valve (34) in branch line (32) opens and closes automatically and synchronously with the valve (15) downstream of flow regulator (14).

## Revendications

1. Procédé de mesure de la pression intra-abdominale par insufflation d'un gaz, en particulier du gaz carbonique, dans la zone intra-abdominale d'un organisme humain ou animal, pour lequel, lors de l'insufflation, le gaz est amené à partir d'un stockage de gaz sous pression par l'intermédiaire d'une ou de plusieurs étapes de stockage intermédiaires et d'au moins un abaissement de la pression, par l'intermédiaire d'une conduite, un régulateur de débit et un outil d'insufflation par exemple une aiguille de Veress, dans la zone intra-abdominale du corps, caractérisé en ce que, après l'abaissement de la pression dans la conduite, la pression présente de gaz et la résistance à l'écoulement de la conduite sont mesurés et que, à partir de la vitesse d'écoulement dans la conduite, à partir de la résistance à l'écoulement et de la pression gazeuse, la pression intra-abdominale est calculée.

2. Dispositif pour l'exécution du procédé selon la revendication 1 avec un magasin de gaz de pression (40) pour le gaz qui doit être insufflé, avec, de préférence, un ou plusieurs magasins intermédiaires (50), avec au moins un réducteur (44) de pression dans lesquels le gaz est amené dans la conduite (10) et dans la zone intra-abdominale et dans un outil d'insufflation (pour 16) par exemple une aiguille de Veress pour lequel dans la conduite (10) entre le réducteur de pression (12) et l'outil d'insufflation (16), un régulateur de débit (14) est prévu pour le gaz ainsi en aval du régulateur de débit (14) un appareil de mesure de pression (22), dont la valeur de mesure est amenée comme une fonction du temps d'une connexion d'exploitation électronique (20), caractérisé en ce que la mesure de valeur présentée dans le branchement d'exploitation (20) $P_{ges}$ de l'appareil de mesure de pression (22) est différencié selon la durée que la pression de résistance de la conduite (10) qui doit être coordonnée au dispositif et à l'outil d'insufflation (16), est la même pression $P_{wid}$ que celle qui est mesurée au débit de l'insufflation au moment où la première dérivation de la valeur de la pression est égale à 0 et que la pression intra-abdominale $P_{abd}$ peut être déterminée par la différence de pression $P_{ges}-P_{wid}$.

3. Dispositif selon la revendication 2, caractérisé en ce que dans la conduite (10) entre le réducteur de pression (12) et l'outil d'insufflation (16), on dispose un appareil de mesure (30) au moins pour la vitesse d'écoulement du gaz.

4. Dispositif selon la revendication 3, caractérisé en ce qu'en aval du régulateur de débit (14), on dispose un clapet manœuvré électroniquement (15) — par exemple un clapet magnétique.

5. Dispositif selon la revendication 3 ou 4, caractérisé en ce que, en aval du régulateur de débit (14), on dispose un équipement de signalisation du débit (26) pour le gaz qui s'écoule dans la conduite (10).

6. Dispositif selon l'une des revendications 3 à 5, caractérisé en ce qu'en aval du régulateur de débit (14) ou de l'équipement de signalisation du débit (26), un clapet de sécurité (28), par exemple un clapet magnétique, est disposé dans la conduite (10).

7. Dispositif selon l'une des revendications 2 à 6, caractérisé en ce qu'en aval du régulateur de débit (14) un appareil de mesure de débit est disposé dans la conduite (10) dont la valeur de mesure est fournie également dans le branchement d'exploitation (20) électronique.

8. Dispositif selon la revendication 7, caractérisé en ce qu'un branchement (32) est disposé, qui rattache dans la conduite (10) un point en amont du régulateur de débit (14) à un point en aval de l'appareil de mesure de débit (30), pour lequel le débit est réglable dans le branchement (32) par un clapet (34) manœuvré électroniquement, par exemple un clapet magnétique.

9. Dispositif selon la revendication 8, caractérisé en ce que le clapet (34) dans le branchement (32) se ferme ou s'ouvre automatiquement et d'une manière synchrone avec le clapet (15) en aval du régulateur de débit (14).

EP 0 169 972 B1